# EUROPEAN PATENT SPECIFICATION

(11) **EP 1 457 230 B1**
(45) Date of publication and mention of the grant of the patent: **17.05.2006**
(21) Application number: 04006116.0
(22) Date of filing: 15.03.2004
(51) Int. Cl.: A61M 25/06, A61M 25/00, A61M 25/01

(54) **Catheter**
Katheter
Cathéter

(30) Priority: 14.03.2003 JP 2003070808
(43) Date of publication of application: 15.09.2004
(73) Proprietor: TERUMO KABUSHIKI KAISHA, Tokyo (JP)
(72) Inventor: Mihara, Nobuaki, Fujinomiya-shi Shizuoka (JP); Mutoh, Makoto, Ohsato-gun Saitama (JP)
(74) Representative: Casalonga, Axel

(56) References cited:
- EP-A- 0 875 263
- WO-A-01/30433
- US-A- 5 290 247
- US-A- 5 395 332
- US-A- 5 449 362
- US-A- 6 010 464
- US-B1- 6 447 501

## Description

### 1. Field of the Invention

The present invention relates to a catheter for penetrating a stenotic lesion or an occluded lesion occurred formed in a lumen in the human body.

### 2. Related Background Art

For example, in the case where stenosis or occlusion is occurred in a lumen in the human body, such as a blood vessel, a bile duct, a trachea, an esophagus, or an urethra, a treatment for opening the stenosis or occlusion to recover the functions of these organs is required. Angioplasty applied to an ischemic heart disease will be described as an example of such a treatment.

Owing to the rapid increase in number of patients of ischemic heart diseases (angina pectoris, myocardial infarct, etc.) due to westernization of dietary habits in Japan, percutaneous transluminal coronary angioplasty (PTCA) is performed as a method for alleviating such diseases and is rapidly spreading. The PTCA is the following procedure. A small incision is formed in an artery of a leg or an arm of a patient, and an introducer sheath (introduction unit) is placed therein. While a guide wire is allowed to travel first through a lumen of the introducer sheath, a long hollow tube called a guide catheter is inserted in a blood vessel, and placed at an entrance of a coronary artery. After that, the guide wire is pulled out, and another guide wire and a balloon catheter are inserted in a lumen of the guide catheter. While the guide wire is allowed to travel first, the balloon catheter is allowed to proceed to a lesion (stenotic lesion part or occluded lesion) of the coronary artery of the patient by visualization with an X-ray. A balloon is positioned in the lesion. A doctor inflates the balloon at that position once or a plurality of times at a predetermined pressure for about 30 to 60 seconds. As a result, the lumen of the blood vessel in the lesion is opened, whereby the amount of blood flowing through the lumen of the blood vessel increases.

However, in the case where the stenosis of a lesion is tight, and the lesion is substantially occluded, a balloon catheter may not be able to pass through the lesion.

Thus, a catheter (for penetrating a coronary artery) for previously penetrating a lesion before inserting a balloon catheter has been developed (e.g., see JP 2002-301161 A). This catheter has a tubular body having a guide wire lumen and a port provided on a proximal end side of the tubular body, and is configured so as to insert a guide wire in the guide wire lumen from the port.

However, according to the catheter described in JP 2002-301161 A, the guide wire lumen is formed over the entire length of the catheter. Therefore, to exchange the catheter with a balloon catheter with the guide wire placed in the blood vessel, it is required that the length of the guide wire be set to be twice or more the entire length of the catheter. When the catheter is pulled out from the blood vessel, it is required that the catheter be operated along such a long guide wire as described above. The requirement results in poor operability when the catheter is exchanged with the balloon catheter.

Furthermore, the catheter described in JP 2002-301161 A is composed of a hollow tubular member over the entire length, so that the catheter is highly soft (flexible) over the entire length. Therefore, a push-in force applied from a hand side (port) is difficult to be transmitted, and the catheter may have difficulty in penetrating stenotic lesion.

Reference can also be made to document US-A-5395332 which discloses a catheter according for the preamble of claim 1 for use in angioplasty comprising a metallic tube having a distal portion more flexible than a proximal portion and a balloon on the distal end of the tube

### SUMMARY OF THE INVENTION

An object of the present invention is to provide a catheter excellent in push-in property, capable of easily and rapidly penetrating a stenotic lesion, and capable of being exchanged with a balloon catheter easily and rapidly.

The above-mentioned object is achieved by the following (1) to (10).
(1) A catheter for penetrating a stenotic lesion occurred in a lumen in a human body, including:
   a linear wire; and
   a tubular body placed on a distal end side of the wire and allowing a guide wire to be inserted through its hollow portion.
(2) The catheter according to the above (1), in which the wire has a metal wire and a covering layer composed of a resin material covering an outside of the metal wire.
(3) The catheter according to the above (1) or (2), in which the wire has a surface layer composed of a hydrophilic material covering an outer surface of the wire.
(4) The catheter according to any one of the above (1) to (3), in which the tubular body includes a plurality of markers each having a visualization property arranged in a longitudinal direction.
(5) The catheter according to any one of the above (1) to (4), in which the tubular body has an inner layer positioned on an inner circumferential side, an outer layer formed on an outer circumferential side of the inner layer, and a reinforcing body placed between the inner layer and the outer layer.
(6) The catheter according to any one of the above (1) to (5), further including an operation portion placed on a proximal end side of the wire.
(7) The catheter according to the above (6), in which the operation portion can be adjusted and fixed for its position with respect to the wire.
(8) The catheter according to the above (6), in which the operation portion is adhered to the wire.
(9) The catheter according to any one of the above (1) to (8), in which the tubular body is placed with its center decentered with respect to a center of the wire.
(10) The catheter according to any one of the above (1) to (9), in which the wire is connected to the tubular body under a condition that a distal end portion of the wire partially overlaps with a proximal end portion of the tubular body.

As described below, the catheter of the present invention has an excellent push-in property. Therefore, a push-in force applied from a proximal end side is transmitted to a distal end portion exactly, and as a result, the catheter can penetrate a stenotic lesion occurred in a lumen in the human body easily and rapidly.

Furthermore, the catheter of the present invention can be exchanged with a balloon catheter even if the length of a guide wire is short. Therefore, the catheter of the present invention can be exchanged with the balloon catheter easily and rapidly.

### BRIEF DESCRIPTION OF THE DRAWINGS

In the accompanying drawings:
FIG. 1 is an entire front view showing an embodiment of a catheter of the present invention;
FIG. 2 is a vertical cross-sectional view showing an enlarged portion on a distal end side of the catheter shown in FIG. 1; and
FIG. 3 is a schematic plan view of an evaluation system for evaluating the catheter of the present invention for push-in property.

### DESCRIPTION OF ILLUSTRATIVE EMBODIMENTS

Hereinafter, a catheter of the present invention will be described in detail by way of preferable embodiments with reference to the attached drawings.

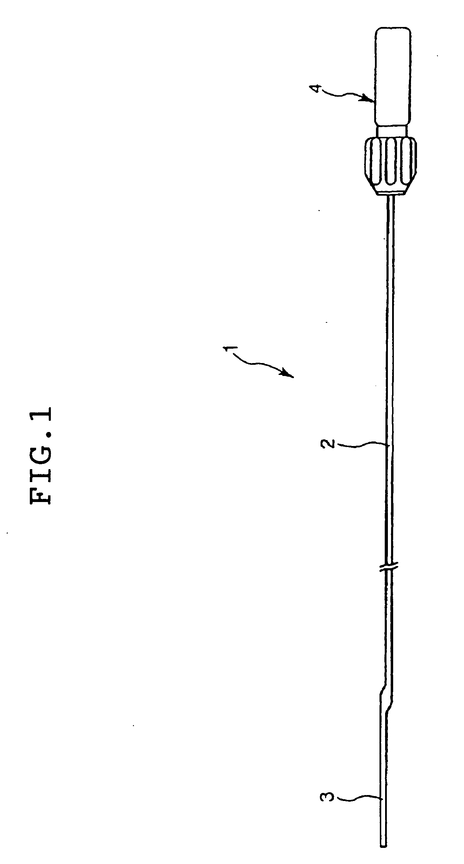

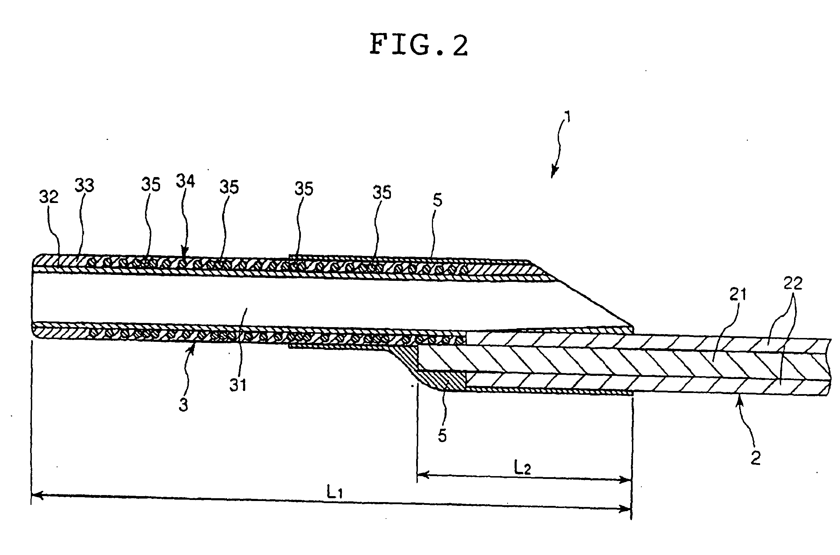

FIG. 1 is an entire front view showing an embodiment of a catheter of the present invention. FIG. 2 is a vertical cross-sectional view showing an enlarged portion on a distal end side of the catheter shown in FIG. 1. In the following description, the left side in each of FIGS. 1 and 2 refers to a "distal end", and the right side in each of FIGS. 1 and 2 refers to a "proximal end".'

A catheter 1 (catheter for penetrating a stenotic lesion) shown in FIGS. 1 and 2 is a catheter for penetrating a stenotic lesion or an occluded lesion (hereinafter, the stenotic lesion and the occluded lesion will be merely referred to as a "stenotic lesion" collectively) occurred in a lumen in the human body, such as a blood vessel, a bile duct, a trachea, an esophagus, or an urethra (hereinafter, referred to as a "blood vessel" as a representative).

As shown in FIGS. 1 and 2, the catheter 1 includes a linear wire 2, a tubular body 3 placed on a distal end side of the wire 2 and allowing a guide wire (not shown) to be inserted in (to penetrate) its hollow portion (lumen) 31, and an operation portion (holding portion) 4 placed on a proximal end portion of the wire 2.

The entire length of the catheter 1 is not particularly limited, but preferably in the range of 900 to 1700 mm, and more preferably in the range of 1100 to 1500 mm.

When a treatment for opening a stenotic lesion by using a balloon catheter (not shown) is performed, in the case where it is difficult for a balloon portion of the balloon catheter to pass through (penetrate) the stenotic lesion owing to the tight stenosis, the catheter 1 is used for facilitating the passage. More specifically, prior to the use of the balloon catheter, the catheter 1 is inserted along a guide wire (not shown) placed in a blood vessel. A push-in force is applied to the catheter 1 so as to allow a distal end portion (tubular body 3) thereof to penetrate the stenotic lesion. After that, while the guide wire is placed in the blood vessel, the catheter 1 is pulled out from the blood vessel and exchanged with the balloon catheter. The balloon catheter is inserted along the guide wire, and a push-in force is applied thereto, whereby the balloon portion is allowed to pass through (penetrate) the stenotic lesion. By using the catheter 1 in this manner, the tubular body 3 of the catheter 1 has penetrated the stenotic lesion once. Therefore, the balloon catheter can be allowed to pass through the stenotic lesion easily thereafter.

As shown in FIG. 2, in the catheter 1, the hollow portion 31, functioning as a guide wire lumen through which a guide wire is inserted, is formed merely in a portion of the tubular body 3 positioned on a distal end side, and in a portion of the wire 2 positioned on a proximal end side with respect to the portion of the tubular body 3, no guide wire lumen is formed. This configuration provides the following two advantages.

The first advantage resides in that the distal end portion (tubular body 3) of the catheter 1 can penetrate (pass through) a stenotic lesion easily and rapidly. The reason for this is as follows. The portion of the wire 2 is solid, so that the wire 2 has relatively high flexural rigidity and torsional rigidity. Therefore, the push-in force applied by an operator from the proximal end side of the catheter 1 is transmitted to the distal end portion of the catheter 1 (tubular body 3) exactly by the wire 2. More specifically, the catheter 1 includes the wire 2 and is thus excellent in push-in property (performance in which a push-in force by an operator can be transmitted exactly from the proximal end side (operation portion 4) to the distal end side of the catheter 1). Thus, the distal end portion (tubular portion 3) of the catheter 1 can be allowed to penetrate (pass through) the stenotic lesion easily and rapidly.

In contrast, unlike the present invention, in the case of a catheter for penetrating a stenotic lesion in which the guide wire lumen is formed so as to extend over the entire length, the catheter is formed of a hollow tubular member over the entire length. Therefore, such a catheter lacks flexural rigidity and torsional rigidity. Consequently, a sufficient push-in property is not obtained, which is likely to cause inconvenience that the catheter cannot penetrate the stenotic lesion easily.

The second advantage of the catheter 1 of the present invention is that an exchange operation (manipulation) for exchanging with a balloon catheter can be performed easily and rapidly. The reason for this is as follows. For pulling out the catheter 1 from a blood vessel, and exchanging it with a balloon catheter, the length of a portion exposed outside the human body of a guide wire placed in the blood vessel only needs to be the length of the tubular body 3. Thus, in the case of using the catheter 1, it is possible to use a guide wire with a relatively short length. Therefore, the catheter 1 and the balloon catheter can be pulled out or inserted along the guide wire easily and rapidly. More specifically, in the catheter 1, an exchange operation for exchanging with the balloon catheter can be performed easily and rapidly.

In contrast, unlike the present invention, in the case of a catheter for penetrating a stenotic lesion in which the guide wire lumen is formed so as to extend over the entire length, for exchanging with the balloon catheter, the length of a portion exposed outside the human body of a guide wire placed in the blood vessel needs to be at least the entire length of the catheter for penetrating a stenotic lesion. More specifically, the entire length of the guide wire needs to be very long, i.e., at least twice the entire length of the catheter for penetrating a stenotic lesion, and the catheter must be exchanged with the balloon catheter along this long guide wire. Thus, it is cumbersome to exchange the catheter with the balloon catheter, and great amounts of time and labor are required.

Hereinafter, the configuration of each portion of the catheter 1 will be described.

As shown in FIG. 2, the wire 2 has a metal wire 21 and a covering layer 22 composed of a resin material, covering the outside of the metal wire 21.

Although a metal material constituting the metal wire 21 is not particularly limited, it is preferable that the metal wire 21 be made of stainless steel (SUS), an Ni-Ti alloy, a cobalt alloy, a connected body thereof (e.g., a coupled (connected) body in which an SUS wire is coupled (connected) with an Ni-Ti alloy wire in the middle in a longitudinal direction), or the like. According to this configuration, the wire 2 is provided with appropriate rigidity (flexural rigidity and torsional rigidity), which enhances a push-in property and transmittance of a torque. Consequently, the catheter 1 can penetrate a stenotic lesion more easily.

Although the resin material constituting the covering layer 22 is not particularly limited, it is preferable that the covering layer 22 be formed of, for example, various kinds of thermoplastic elastomers such as a polyurethane elastomer, a polyester elastomer, and a polyamide elastomer, or a mixture thereof. Of those, a polyurethane elastomer is more preferable. In the case where the covering layer 22 is formed of a polyurethane elastomer, there is an advantage in that the covering layer 22 is particularly excellent in thermal processability.

Furthermore, the covering layer 22 may contain, for example, an X-ray non-transparent material (x-ray visualization agent) such as tungsten.

It is preferable that a surface layer composed of a hydrophilic material (hydrophilic polymer) be provided on an outer surface (outer surface of the covering layer 22) of the wire 2. According to this configuration, the catheter 1 can be inserted more smoothly and more easily. Although the hydrophilic material is not particularly limited, examples of the hydrophilic material include a copolymer of methyl vinyl ether and maleic anhydride and a copolymer of dimethylacrylamide and glycidyl methacrylate.

According to one preferable aspect, the wire 2 is composed of: a long proximal portion extending from its proximal end portion and having a substantially constant outer diameter; and a distal portion connected to the distal end side of the proximal portion with its outer diameter decreasing continuously toward the distal end.

The outer diameter of the wire 2 in the proximal portion is not particularly limited. Although a preferable value of the outer diameter varies depending upon the constituent material and the purpose of use, generally, the outer diameter is preferably 0.5 to 1.5 mm, and more preferably 0.8 to 1.1 mm. Furthermore, the length of the distal portion is preferably 30 to 150 mm, and more preferably 50 to 100 mm. Furthermore, the outer diameter of the distal end of the wire 2 is preferably 0.4 to 1.4 mm, and more preferably 0.7 to 1.0 mm.

The tubular body 3 is provided on the distal end side of the wire 2. The hollow portion 31 of the tubular body 3 is opened to the distal end and the proximal end of the tubular body 3, whereby a guide wire can be inserted (penetrate) in the hollow portion 31.

The tubular body 3 has an inner layer 32 positioned on an inner circumferential side, an outer layer 33 formed on an outer circumferential side of the inner layer 32, and a reinforcing body (reinforcing member) 34 placed between the inner layer 32 and the outer layer 33.

Although the constituent material for the inner layer 32 is not particularly limited, for example, the inner layer 32 is preferably formed of a fluorine resin such as polytetrafluoroethylene (PTFE). According to this configuration, the friction coefficient of the inner circumferential surface of the inner layer 32 becomes small. Therefore, the sliding resistance between the inner circumferential surface of the inner layer 32 and the guide wire decreases, whereby the guide wire can be inserted more smoothly.

Although the constituent material for the outer layer 33 is not particularly limited, for example, it is preferable that the outer layer 33 be composed of various kinds of thermoplastic elastomers such as a polyurethane elastomer, a polyester elastomer, and a polyamide elastomer, or a mixture thereof. Furthermore, the outer layer 33 may be configured by combining (coupling) a plurality of tubes having different conditions with respect to a hardness and an outer diameter and the like with each other.

In this embodiment, the reinforcing body 34 is a spiral coil composed of tungsten. The reinforcing body 34 is placed in such a manner that the reinforcing body 34 is buried in the outer layer 33 (or the inner layer 32). The reinforcing body 34 is not limited to a spiral coil, and may be a braided body (net-shaped body), a bar-shaped body, or the like. Its material is not limited to tungsten. The reinforcing body 34 may be made of stainless steel or the like.

The outer surface of the tubular body 3 is preferably provided with a surface layer composed of a hydrophilic material (hydrophilic polymer). According to this configuration, the catheter 1 can be inserted more smoothly and more easily. The same materials as those described above can be used as the hydrophilic material.

Although the outer diameter of the tubular body 3 is not particularly limited, the outer diameter is preferably 0.5 to 1.5 mm, and more preferably 0.7 to 1.0 mm. Furthermore, the outer diameter of the tubular body 3 may vary in a longitudinal direction. For example, the outer diameter may decrease gradually toward a distal end direction. Furthermore, the outer diameter of the tubular body 3 in a fixed portion with the wire 2 is preferably 0.8 to 1.5 mm, and more preferably 1.0 to 1.3 mm.

Although the inner diameter of the tubular body 3, in other words, the diameter of the hollow portion 31, is not particularly limited, the inner diameter is preferably 0.4 to 0.8 mm, and more preferably 0.45 to 0.65 mm.

Although the length of the tubular body 3 (length represented by L₁ in FIG. 2) is not particularly limited, the length is preferably in the range of 100 to 400 mm, and more preferably 200 to 300 mm. Setting the length of the tubular body 3 to be within such a range can provide excellent followingness when the catheter 1 is inserted in a blood vessel, which is bent in a complicated manner, along the guide wire, and can sufficiently shorten the length of the guide wire required for exchanging with the balloon catheter. As a result, an exchange operation can be performed more easily and more rapidly.

The tubular body 3 has a plurality of markers 35 each having an X-ray visualization property (X-ray non-transparency). Those markers 35 are arranged at intervals in the longitudinal direction of the tubular body 3. With this arrangement, when the tubular body 3 is allowed to penetrate a stenotic lesion of the blood vessel under X-ray radioscopy, the markers 35 function as a scale, whereby the length of the stenotic lesion can be measured (identified). Although the set interval (pitch) of the markers 35 is not particularly limited, it is preferably 5 to 15 mm, and more preferably about 10 mm.

In this embodiment, those markers 35 are configured by closely winding the reinforcing body 34 composed of a spiral coil at several portions. This makes it unnecessary to provide another member as the markers 35, so that the catheter 1 can be produced easily, and the tubular body 3 can have a decreased diameter.

The markers 35 each have an X-ray visualization property under X-ray radioscopy owing to X-ray non-transparency. Such markers 35 usually have visualization properties even in CT scanning, MRI, and the like, so that they can be used even in CT scanning, MRI, and the like.

The tubular body 3 and the wire 2 are coupled (fixed) under a condition that the distal end portion of the wire 2 and the proximal end portion of the tubular body 3 partially overlap with each other in a longitudinal direction. With this configuration, the wire 2 and the tubular body 3 overlap with each other in the coupled portion (fixed portion). Therefore, high coupling strength can be obtained, and the enlargement of the distal end portion of the catheter 1 can be prevented.

Although a method for fixing the wire 2 and the tubular body 3 is not particularly limited, they are fixed by covering the outside (outer circumference) of the overlapped portion between the wire 2 and the tubular body 3 with a reinforcing tube (coupling member) 5 in this embodiment. In particular, in the case where the covering layer 22 of the wire 2, the outer layer 33 of the tubular body 3, and the reinforcing tube 5 are made of the same or similar material (e.g., a polyurethane elastomer), the overlapped portion between the wire 2 and the tubular body 3 is covered with the reinforcing tube 5, and thereafter, they are fused, whereby the wire 2 and the tubular body 3 can be fixed more strongly in an easy process.

Although the length (length represented by L₂ in FIG. 2) of the overlapped portion between the wire 2 and the tubular body 3 is not particularly limited, it is preferably 1 to 100 mm, and more preferably 5 to 60 mm.

The tubular body 3 is provided with its center decentered with respect to the center of the wire 2. With this configuration, the hollow portion 31 can be kept wide and straight in the vicinity of the fixed portion between the tubular body 3 and the wire 2. Therefore, the guide wire can be inserted more smoothly.

The operation portion 4 is provided at the proximal end portion of the wire 2. An operator grabs the operation portion 4, thereby more easily operating (pushing, twisting, etc.) the catheter 1.

The operation portion 4 may be fixed to the proximal end portion of the wire 2. Alternatively, the operation portion 4 may be adjusted and fixed for its position at an arbitrary position with respect to the wire 2 in a longitudinal direction. This configuration allows the operation portion 4 to be adjusted to be an easy-to-handle position. Any configuration for enabling the operation portion 4 to be fixed at an arbitrary position of the wire 2 may be used. Examples of the configuration include a configuration similar to an operation holding member of a guide wire described in JP 5-29543 U.

The embodiment of the catheter according to the present invention shown in FIGS. 1 and 2 has been described above. However, the present invention is not limited thereto. Each portion constituting the catheter can be replaced by an arbitrary configuration capable of exhibiting the similar function. Furthermore, an arbitrary component may be added.

This application claims priority on Japanese patent application No.2003-70808, the contents of which are hereby incorporated by reference. In addition, the contents of literatures cited herein are incorporated by reference.

### Examples

Hereinafter, the present invention will be described specifically by way of examples. It should be noted that the present invention is not limited thereto.

### (Example 1)

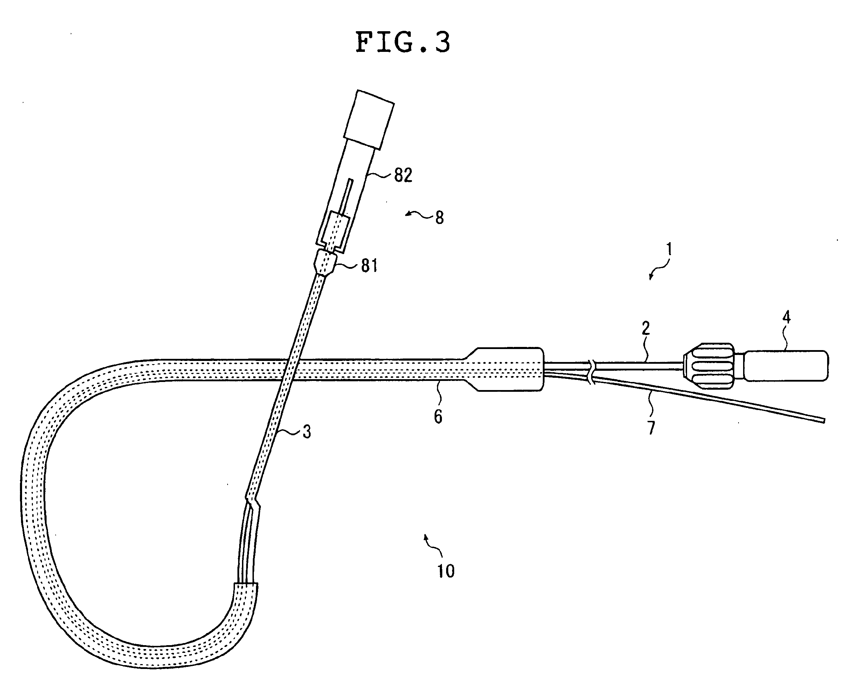

To evaluate the catheter of the present invention for push-in property, an evaluation system was produced. FIG. 3 is a schematic plan view showing the evaluation system for evaluating the catheter of the present invention for push-in property. The evaluation system 10 shown in FIG. 3 is composed of the catheter 1 of the present invention, a guiding catheter 6, a guide wire 7, and a load detecting portion 8 having a torque device 81 and a load sensor 82. In FIG. 3, for convenience of the description, the catheter 1 is shown with the thickness thereof and the like enlarged.

The catheter 1 of the present invention used for evaluation was the same as that shown in FIGS. 1 and 2 (provided that the distal end side of the wire 2 was tapered in this example). The size and material of each portion are as follows.

### <Size>

Length of the wire 2: 1060 mm
Length of L₂: 10 mm
Length of L₁: 250 mm
Outer diameter of the covering layer 22 of a portion between 0 and 980 mm from the proximal end side of the wire 2: 0.92 mm
Outer diameter of the metal wire 21 of a portion between 0 and 980 mm from the proximal end side of the wire 2: 0.60 mm
Outer diameter of the covering layer 22 of a portion between 980 and 1050 mm from the proximal end side of the wire 2: tapered from 0.92 mm (980 mm from the proximal end side) to 0.82 mm (1050 mm from the proximal end side)
Outer diameter of the metal wire 21 of a portion between 980 and 1050 mm from the proximal end side of the wire 2: tapered from 0.60 mm (980 mm from the proximal end side) to 0.35 mm (1050 mm from the proximal end side)
Outer diameter of a portion (corresponding to L₂) between 1050 and 1060 mm from the proximal end side of the wire 2: 1.21 mm
Outer diameter of a portion between 0 and 90 mm from the proximal end side of the tubular body 3: 0.87 mm
Outer diameter of a portion between 90 and 250 mm from the proximal end side of the tubular body 3: 0.83 mm
Inner diameter of the tubular body 3: 0.56 mm

### <Material>

Metal wire 21: Ni-Ti
Covering layer 22: polyurethane elastomer containing 45 wt% of tungsten
Inner layer 32: PTFE
Portion between 0 and 90 mm from the proximal end side of the outer layer 33: polyurethane elastomer
Portion between 90 and 250 mm from the proximal end side of the outer layer 33: polyester elastomer
Reinforcing body 34: spiral coil composed of tungsten
Reinforcing tube 5: polyurethane elastomer

Provided on an outer circumferential surface of a portion between 0 and 780 mm from the distal end side of the catheter 1 is a hydrophilic coating of a dimethylacrylamide-glycidyl methacrylate copolymer.

First, the guiding catheter 6 (Heart Rail 6, produced by Terumo Corp.; having an inner diameter of 1.8 mm and a length of 100 cm) primed with distilled water was bent in a shape as shown in FIG. 3 to produce a blood vessel model. The distal end of the guiding catheter 6 was placed at a position that was assumed to be engaged with an entrance of the coronary artery.

Then, the guide wire 7 (Cross Wire Ex 7, produced by Terumo Corp.: outer diameter: 0.36 mm; length: 180 cm) was inserted in the tubular body 3 of the above-described catheter 1. After that, the catheter 1 was inserted in the guiding catheter 6 together with the guide wire 7.

Furthermore, the load detecting portion 8 was set so that the guide wire 7 was inserted through the torque device 81 and the distal end of the tubular body 3 of the catheter 1 came into contact with the end of the torque device 81. Then, the guide wire 7 was fixed on a proximal end side.

The catheter 1 was guided with a guide tube (inner diameter: 2 mm) (not shown) between the distal end of the guiding catheter 6 and the torque device 81. The guide tube was provided so as not to come into contact with the torque device 81.

After that, the operation portion 4 of the catheter 1 was pushed in at a distance of 10 mm or 15 mm. The load (striking resistance) detected by the load sensor 82 of the load detecting portion 8 was measured through the distal end of the tubular body 3 and the torque device 81.

Table 1 shows the results.

### (Comparative Example 1)

The striking resistance was measured in the same way as in Example 1 except for using a catheter (NAVI-CATH produced by Terumo Corp.) with a guide wire lumen formed over the entire length of the catheter in place of the catheter 1 of the present invention.

### Table 1 shows the results.

**Table 1**

| | Striking Resistance (gf) | |
|---|---|---|
| | 10mm | 15mm |
| Example 1 | 23 | 22 |
| Comparative Example 1 | 15 | 14 |

As shown in Table 1, it was confirmed that the catheter of the present invention (Example 1) has a high striking resistance and an excellent push-in property, compared with the catheter with the guide wire lumen formed over the entire length of the catheter (Comparative Example).

## Claims

1. A catheter for penetrating a stenotic lesion occurred in a lumen in a human body, including:
a linear wire (2), and
a tubular body (3) placed on a distal end side of the wire and allowing a guide wire to be inserted through its hollow portion, **characterized in that** the wire is a metal wire and has a covering layer (22) composed of a resin material covering an outside of the metal wire.

2. The catheter according to claim 1, in which the wire has a surface layer composed of a hydrophilic material covering an outer surface of the wire.

3. The catheter according to claim 1 to 2, in which the tubular body (3) includes a plurality of markers (35) each having a visualization property arranged in a longitudinal direction.

4. The catheter according to any one of claims 1 to 3, in which the tubular body has an inner layer positioned on an inner circumferential side, an outer layer formed on an outer circumferential side of the inner layer, and a reinforcing body placed between the inner layer and the outer layer.

5. The catheter according to any one of claims 1 to 4, further including an operation portion placed on a proximal end side of the wire (2).

6. The catheter according to claim 5, in which the operation portion can be adjusted and fixed for its position with respect to the wire (2).

7. The catheter according to claim 5, in which the operation portion is adhered to the wire (2).

8. The catheter according to any one of claims 1 to 7, in which the tubular body is placed with its center decentered with respect to a center of the wire (2).

9. The catheter according to any one of claims 1 to 8, in which the wire (2) is connected to the tubular body (3) under a condition that a distal end portion of the wire partially overlaps with a proximal end portion of the tubular body.

## Patentansprüche

1. Katheter zum Durchdringen einer stenotischen Läsion, die in einem Lumen in einem menschlichen Körper aufgetreten ist, umfassend:
einen linearen Draht (2), und
einen röhrenförmigen Körper (3), der auf einer distalen Endseite des Drahts platziert ist und einem Führungsdraht gestattet, durch seinen hohlen Abschnitt eingeführt zu werden, **dadurch gekennzeichnet, dass** der Draht ein Metalldraht ist und eine Überzugsschicht (22) aufweist, die aus einem Harzmaterial besteht, das eine Außenseite des Metalldrahts überzieht.

2. Katheter nach Anspruch 1, bei welchem der Draht eine Oberflächenschicht aufweist, die aus einem hydrophilen Material besteht, das eine äußere Oberfläche des Drahts überzieht.

3. Katheter nach Anspruch 1 bis 2, bei welchem der röhrenförmige Körper (3) eine Mehrzahl von Markierungen (35) umfasst, die jeweils eine in einer Längsrichtung angeordnete sichtbar machende Eigenschaft besitzen.

4. Katheter nach einem der Ansprüche 1 bis 3, bei welchem der röhrenförmige Körper eine innere Schicht aufweist, die auf einer innen umlaufenden Seite angeordnet ist, eine äußere Schicht, die auf einer außen umlaufenden Seite der inneren Schicht gebildet wird, und einen Verstärkungskörper, der zwischen der inneren Schicht und der äußeren Schicht platziert ist.

5. Katheter nach einem der Ansprüche 1 bis 4, der weiterhin einen Bedienungsabschnitt umfasst, der auf einer proximalen Endseite des Drahts (2) angeordnet ist.

6. Katheter nach Anspruch 5, bei welchem der Bedienungsabschnitt reguliert und in seiner Position mit Bezug auf den Draht (2) befestigt werden kann.

7. Katheter nach Anspruch 5, bei welchem der Bedienungsabschnitt mit dem Draht (2) fest verbunden ist.

8. Katheter nach einem der Ansprüche 1 bis 7, bei welchem die Mitte des röhrenförmigen Körpers im Bezug auf eine Mitte des Drahts (2) dezentriert angeordnet ist.

9. Katheter nach einem der Ansprüche 1 bis 8, bei welchem der Draht (2) mit dem röhrenförmigen Körper (3) unter der Bedingung verbunden ist, dass ein distaler Endabschnitt des Drahts sich teilweise mit einem proximalen Endabschnitt des röhrenförmigen Körpers überlappt.

## Revendications

1. Cathéter permettant de pénétrer une lésion sténotique formée dans une lumière d'un corps humain, comprenant :
un fil linéaire (2), et
un corps tubulaire (3) placé sur un côté d'extrémité distale du fil et permettant à un fil de guidage d'être inséré dans sa partie creuse, **caractérisé en ce que** le fil est un fil métallique et comporte une couche de revêtement (22) composée d'un matériau de type résine qui recouvre une partie extérieure du fil métallique.

2. Cathéter selon la revendication 1, dans lequel le fil comporte une couche de surface composée d'un matériau hydrophile qui recouvre une surface extérieure du fil.

3. Cathéter selon la revendication 1 ou 2, dans lequel le corps tubulaire (3) comprend une pluralité de repères (35) ayant chacun une propriété de visualisation agencée dans une direction longitudinale.

4. Cathéter selon l'une quelconque des revendications 1 à 3, dans lequel le corps tubulaire comporte une couche intérieure positionnée sur un côté périphérique intérieur, une couche extérieure formée sur un côté périphérique extérieur de la couche intérieure, et un corps de renforcement placé entre la couche intérieure et la couche extérieure.

5. Cathéter selon l'une quelconque des revendications 1 à 4, comprenant en outre une partie opérante placée sur un côté d'extrémité proximale du fil (2).

6. Cathéter selon la revendication 5, dans lequel la partie opérante peut être réglée et fixée en position par rapport au fil (2).

7. Cathéter selon la revendication 5, dans lequel la partie opérante adhère au fil (2).

8. Cathéter selon l'une quelconque des revendications 1 à 7, dans lequel le centre du corps tubulaire est décentré par rapport au centre du fil (2).

9. Cathéter selon l'une quelconque des revendications 1 à 8, dans lequel le fil (2) est connecté au corps tubulaire (3) dans un état dans lequel une partie d'extrémité distale du fil et une partie d'extrémité proximale du corps tubulaire se chevauchent partiellement.
